## Europäisches Patentamt
## European Patent Office
## Office européen des brevets

(19)

(11) Numéro de publication: **0 064 924**
**B1**

(12)
# FASCICULE DE BREVET EUROPEEN

(45) Date de publication du fascicule du brevet:
**19.06.85**

(21) Numéro de dépôt: **82400822.1**

(22) Date de dépôt: **04.05.82**

(51) Int. Cl.⁴: **C 07 D 307/60,** C 08 F 8/46,
C 08 F 10/00, C 08 F 110/04,
C 08 F 210/00

(54) **Procédé de préparation d'anhydrides d'acides alcényl-dicarboxyliques.**

(30) Priorité: **11.05.81 FR 8109473**

(43) Date de publication de la demande:
**17.11.82 Bulletin 82/46**

(45) Mention de la délivrance du brevet:
**19.06.85 Bulletin 85/25**

(84) Etats contractants désignés:
**BE DE GB IT NL**

(56) Documents cités:
**US - A - 3 954 812**
**US - A - 4 086 251**

(73) Titulaire: **INSTITUT FRANCAIS DU PETROLE, 4, Avenue de Bois-Préau, F-92502 Rueil-Malmaison (FR)**

(72) Inventeur: **Weill, Jérôme, 16, route des Tourelles, F-69005 Lyon (FR)**
Inventeur: **Garapon, Jacques, 64, rue des Aqueducs, F-69005 Lyon (FR)**
Inventeur: **Sillion, Bernard, 13, rue des Erables, F-78150 Rocquencourt (FR)**

BUNDESDRUCKEREI BERLIN

## Description

L'invention concerne un procédé de préparation d'anhydrides d'acides alcényl-dicarboxyliques.

Les composés alcényl-dicarboxyliques, acides ou anhydrides, sont des matières importantes dans l'indusrie des additifs pour carburants et lubrifiants. Les produits généralement utilisés sont constitués d'une chaine hydrocarbonée de masse comprise entre 200 et 2000 sur laquelle est fixée au moins un groupe succinique.

Des procédés de préparation à partir d'un oligomère d'oléfines et d'anhydride maléique sont connus dans l'art antérieur. La condensation implique une température supérieure à 200°C et la réaction doit être de longue durée pour atteindre une conversion de l'ordre de 75%. Des procédés de ce type sont décrits notamment dans les brevets américains 2 628 942 et 2 634 256.

Afin d'améliorer la conversion, il a été recommandé (brevet français 2 257 604) d'utiliser l'anhydride maléique en excès, mais, même dans ce cas, la température de condensation reste supérieure à 200°C, la durée de la réaction est de l'ordre de 20 heures, et le procédé nécessite une étape d'élimination de l'anhydride maléique excédentaire en fin de réaction.

Il a été ensuite proposé de préparer des composés de même nature par condensation de l'anhydride maléique sur une polyoléfine préalablement chlorée et contenant environ un atome de chlore par molécule de polyoléfine. Une telle technique est décrite par exemple dans le brevet britannique 922 831. La durée de réaction est plus courte et la température à laquelle on doit opérer est moins élevée que dans les cas de condensation de la polyoléfine elle même. De plus, on évite ainsi les réactions secondaires qui se traduisent par la formation de résines polymériques. Il est d'autre part signalé que les anhydrides insaturés se décomposent à partir de 150°C.

On a cherché à simplifier un tel procédé, en particulier en opérant la chloration et la condensation en une seule étape: par exemple le brevet britannique 949 981 décrit la chloration d'une mélange de polyisobutène et d'anhydride maléique à une température de 190 à 210°C, la durée de l'opération de condensation étant, dans ces conditions, de 10 heures.

Le brevet britannique 1 356 802 décrit l'addition d'iode au mélange réactionnel après 25% de conversion: néanmoins, dans ces conditions la température de réaction est de 215°C et il faut maintenir la réaction pendant 24 heures pour obtenir des conversions intéressantes.

Le brevet français 2 273 014 montre que l'on peut utiliser le brome pour catalyser la condensation, mais la température de réaction est de 240°C dans les conditions décrites.

Dans certains brevets, les halogènes utilisés pour catalyser la réaction des polyoléfines sur l'anhydride maléique ne sont pas utilisés sous forme libre mais combinés à d'autres molécules organiques; par exemple, le brevet américain 3 927 041 décrit l'emploi de dibromo 1,3-dialkyl 5,5-hydantoïne et le brevet américain 3 954 812 montre que l'on peut employer les chlorures d'acides, les chloro-acides ou le N-bromosuccinimide, comme agents générateurs d'halogène.

Le mécanisme d'action des dérivés halogénés n'est pas parfaitement expliqué, mais il est vraisemblable que l'on assiste à une réaction d'halogénation de la polyoléfine suivi d'une deshydrohalogénation précédant la condensation sur l'anhydride: c'est d'ailleurs ce schéma que décrit le brevet américain 3 864 270, dans lequel on montre que la polyoléfine peut être chlorée entre 20°C et 150°C, puis déshydrochlorée entre 150°C et 250°C, de préférence à 235°C pendant 5 heures. Après ces opérations, l'anhydride maléique est condensé pendant 6 heures vers 100°C.

On voit clairement que l'étape de déshydrochloration est l'étape qui nécessite la température la plus élevée.

Or, il est maintenant trouvé qu'il est possible de provoquer la déshydrochloration de polyoléfines chlorées dans des conditions douces en utilisant de catalyseurs organiques. L'invention repose sur le fait que ces catalyseurs sont efficaces en présence d'anhydride maléique, et par conséquent, leur utilisation lors de la condensation d'une polyoléfine halogénée sur un anhydride cyclique insaturé conduit à un procédé simplifié de préparation des anhydrides alcényl-dicarboxyliques.

L'intérêt du procédé catalytique de l'invention est qu'il permet de travailler à une température inférieure à celle utilisée dans l'art antérieur, qu'il assure une transformation à une vitesse accrue et conduit à un rendement supérieur en anhydride alcényl-dicarboxylique.

D'une manière générale, le procédé de l'invention comprend donc la mise en réaction d'au moins une polyoléfine halogénée avec une proportion sensiblement stoechiométrique d'au moins un anhydride d'acide dicarboxylique insaturé, en présence d'une proportion mineure d'un catalyseur organique tel que défini ci-dessous. On peut opérer à une température de 150 à 200°C.

Les catalyseurs organiques utilisés dans le procédé de l'invention (qui favorisent la déshydrochloration des polyoléfines chlorées sont des composés qui contiennent une double dont la densité électronique est plus faible que celle des oléfines classiques, ces doubles liaisons étant substituées par des groupements attracteurs d'électrons. Ces composés sont choisis, de plus, parmi ceux qui réagissent difficilement dans la réaction de diène synthèse.

Les composés qui présentent un effet sur la déshydrohalogénation des polyoléfines halogénées sont: les halogéno-quinones, les cyanoquinones, les anhydrides halogéno-maléiques, et les polycyano-oléfines. Parmi tous ces composés, l'anhydride dichloro-maléique s'est mon tré l'un des plus avantageux.

Le catalyseur peut être utilisé en une proportion de 0,5% à 5% en moles. La température de déshydrohalogénation effectuée selon la technique de l'invention est de préférence de 170°C à 185°C.

La réaction est quasi-totale après un temps d'environ 7 à 10 heures. Les polyoléfines halogénés utilisables dans le cadre de la présente invention sont obtenues par halogénation des polyoléfines de type polyisobutène ou polypropylène pour lesquelles la masse moléculaire est comprise entre 200 et 3000, le taux d'halogénation est préférentiellement compris entre 0,8 et 1,5 atome d'halogène par molécule de polyoléfine. L'halogène utilisé peut être le chlore, le brome ou l'iode; pour des raisons économique, on utilisera de préférence le chlore.

Enfin, parmi les anhydrides d'acides dicarboxyliques insaturés, le plus fréquemment employé est l'anhydride maléique.

Les exemples suivants illustrent l'invention.

### Exemple 1

#### Préparation de polyisobutène chloré

5 moles (4775 g) de polyisobutène chloré de masse moléculaire moyenne 955 sont préparées en faisant passer 5 moles (355 g) de chlore gazeux dans 5 moles (4600 g) de polyisobutène de masse moléculaire moyenne 920, à 80°C, à l'abri de la lumière. Le polyisobutène chloré ainsi obtenu contient 3,6% en poids de chlore ce qui correspond globalement à environ un atome de chlore par molécule de polymère.

### Exemple 2

#### Essai comparatif

1 mole (955 g) du polyisobutène chloré ainsi obtenu est chauffée à 180°C. Un barbotage continu d'azote permet de doser l'acide chlorhydrique libéré. Après 1 heure, 7,5% de la quantité théorique maximale sont dosés et, après 7 heures, la réaction est terminée et seulement 34% sont dosés, ce qui indique que les types de chlores spontanément libérables à cette température représentent 34% de la quantité totale de chlore présente dans le polyisobutène chloré.

### Exemple 3

#### Essai comparatif

La même expérience est reprise en chauffant, toujours à 180°C, 1 mole (955 g) du même polyisobutène chloré, en présence de 1,1 mole (108 g) d'anhydride maléique. En 1 heure, 24% de la quantité théorique maximale d'acide chlorhydrique sont dosés et après 8 heures, la réaction est terminée avec seulement 50% de la quantité maximale dosés, ce qui indique que les types de chlores libérables à cette température en présence d'anhydride maléique représentent 50% de la quantité totale de chlore. La quantité d'anhydride alcényl-dicarboxylique formée après cette réaction est de 0,45 mole (rendement: 45% moles).

### Exemple 4

#### Mise en évidence de l'effet de l'anhydride dichloromaléique

La même expérience est reprise en chauffant, toujours à 180°C, 1 mole (955 g) du même polyisobutène chloré, en présence de 1,1 mole (184 g) d'anhydride dichloromaléique. En 1 heure, 80% de la quantité théorique maximale d'acide chlorhydrique sont dosés et après 3 heures, la réaction est terminée et 100% sont dosés; ce qui indique que tous les types de chlores présents dans le polyisobutène chloré sont libérables à cette température en présence d'anhydride dichloromaléique.

### Exemple 5

#### Effet d'une proporotion réduite d'anhydride dichloromaléique

La même expérience est reprise en chauffant, toujours à 180°C, 1 mole (955 g) du même polyisobutène chloré en présence de 0,055 mole (9 g) d'anhydride dichloromaléique. Après 1 heure, 30% de la quantité théorique maximale d'acide chlorhydrique sont dosés, et après 10 heures, la réaction est terminée et 95% de la quantité théorique sont dosés, ce qui indique qu'une très faible quantité d'anhydride dichloromaléique permet la libération de tous les types de chlore. La quantité d'anhydride alcényl-dicarboxylique alors formé est inférieure à 0,005 mole, ce qui montre que l'anhydride dichloromaléique est peu susceptible de réagir ultérieurement avec le polyisobutène chloré déshydrochloré.

### Exemple 6

#### Préparation d'anhydride alcényl-dicarboxylique selon l'invention

La même expérience est reprise en chauffant, toujours à 180°C, 1 mole (955 g) du même polyisobutène chloré en présence de 1,1 mole (108 g) d'anhydride maléique et de 0,055 mole (9 g) d'anhydride dichloromaléique. En 1 heure, 37% de la quantité théorique maximale d'acide chlorhydrique sont dosés, et après 7 heures, la réaction est terminée et 95% sont dosés. La quantité, d'anhydride alcényl-dicarboxylique est alors de 0,91 mole (rendement: 91% en moles).

## Exemple 7

### Préparation d'anhydride alcényl-dicarboxylique selon l'invention

La même expérience est reprise en chauffant, toujours à 180°C, 1 mole (955 g) du même polyisobutène chloré en présence de 1,1 mole (108 g) d'anhydride maléique et de 0,0055 mole (0,9 g) d'anhydride dichloromaléique. En 1 heure, 26% de la quantité théorique maximale d'acide chlorhydrique sont dosés, et après 7 heures, 55% dosés. La quantité d'anhydride alcényl-dicarboxylique est alors de 0,50 mole (rendement: 50% en moles).

## Exemple 8

### Préparation d'anhydride alcényl-dicarboxylique selon l'invention

La même expérience est reprise en chauffant, toujours à 180°C, 1 mole (955 g) du même polyisobutène chloré en présence de 1,1 mole (108 g) d'anhydride maléique et de 0,011 mole (1,8 g) d'anhydride dichloromaléique. En 1 heure, 27% de la quantité théorique maximale d'acide chlorhydrique sont dosés, et après 7 heures, 62% sont dosés. La quantité d'anhydride alcényl-dicarboxylique est alors de 0,59 mole (rendement: 59% en moles).

## Exemple 9

### Préparation d'anhydride alcényl-dicarboxylique selon l'invention

La même expérience est reprise en chauffant, toujours à 180°C, 1 mole du même polyisobutène chloré (955 g) en présence de 1,1 mole (108 g) d'anhydride maléique et de 0,033 mole (5,4 g) d'anhydride dichloromaléique. En 1 heure, 30% de la quantité théorique maximale d'acide chlorhydrique sont dosés, et après 7 heures, 75% sont dosés. La quantité d'anhydride alcényl-dicarboxylique est alors de 0,71 mole (rendement: 71% en moles).

## Exemple 10

### Préparation d'anhydride alcényl-dicarboxyliques selon l'invention

La même expérience est reprise en chauffant, toujours à 180°C, 1 mole du même polyisobutène chloré (955 g) en présence de 1,1 mole (108 g) d'anhydride maléique et de 0,055 mole (13,5 g) d'ortho tétra-chloroquinone. En 1 heure, 28% de la quantité théorique maximale d'acide chlorhydrique sont dosés, et après 7 heures, 65% sont dosés. La quantité d'anhydride alcényl-dicarboxylique est alors de 0,62 mole (rendement 62% en moles).

## Revendications

1. Procédé de préparation d'anhydride d'acide alcényl-dicarboxylique, dans lequel on fait réagir au moins une polyoléfine halogénée et au moins un anhydride d'acide dicarboxylique insaturé en proportions sensiblement stoechiométrique, caractérisé en ce que l'on opère en présence d'une proportion mineure d'au moins un composé organique choisi parmi les halogéno-quinones, les cyano-quinones, les anhydrides halogéno-maléiques et les polycyanooléfines, à une température de 150 à 200°C.

2. Procédé selon la revendication 1, caractérisé en ce que ledit composé organique consiste en l'anhydride dichloro-maléique.

3. Procédé selon l'une des revendications 1 et 2, caractérisé en ce que ledit composé organique est utilisé en une proportion molaire de 0,5 à 5% par rapport à la quantité d'anhydride d'acide dicarboxylique insaturé mise en jeu.

4. Procédé selon l'une des revendications 1 à 3, caractérisé en ce que l'on opère à une température de 170 à 185°C.

5. Procédé selon l'une des revendications 1 à 4, caractérisé en ce que ladite polyoléfine halogénée est obtenue par halogénation par du chlore, du brome ou de l'iode d'une polyoléfine de masse moléculaire de 200 à 3000, le taux d'halogénation étant d'environ 0,8 à 1,5 atome d'halogène par molécule de polyoléfine.

6. Procédé selon la revendication 5, caractérisé en ce que ladite polyoléfine est choise parmi les polyisobutène et les poly-propylènes.

7. Procédé selon l'une des revendications 5 et 6, caractérisé en ce que ladite polyoléfine halogénée consiste en un polyisobutène chloré.

8. Procédé selon l'une des revendications 1 à 7, caractérisé en ce que ledit anhydride d'acide dicarboxylique insaturé consiste en l'anhydride maléique.

## Patentansprüche

1. Verfahren zur Herstellung von Alkenyldicarbonsäureanhydriden, wobei man mindestens ein halogeniertes Polyolefin und mindestens ein Anhydrid einer ungesättigten Dicarbonsäure, in praktisch stöchiometrischen Verhältnissen miteinander reagieren läßt, dadurch gekennzeichnet, daß man in Gegenwart einer kleineren Menge von zumindest einer organischen Verbindung, die aus Halogenchinonen, Cyanochinonen, Halogenmaleinsäureanhydriden und Polycyanoolefinen gewählt ist, bei einer Temperatur von 150°C bis 200°C arbeitet.

2. Verfahren nach Anspruch 1, dadurch gekennzeichnet, daß die organische Verbindung aus Dichlormaleinsäureanhydriden besteht.

3. Verfahren nach einem der Ansprüche 1 und 2, dadurch gekennzeichnet, daß die organische Verbindung in einem molaren Verhältnis von 0,5 bis 5%, bezogen auf die Menge des eingesetzten ungesättigten Carbonsäureanhydrids verwendet wird.

4. Verfahren nach einem der Ansprüche 1 bis 3, dadurch gekennzeichnet, daß man bei einer Temperatur von 170° C bis 185° C arbeitet.

5. Verfahren nach einem der Ansprüche 1 bis 4, dadurch gekennzeichnet, daß das halogenierte Polyolefin durch Halogenieren eines Polyolefins von der Molekularmasse 200 bis 3000 mit Chlor, Brom oder Jod erhalten ist, wobei der Halogenierungsgrad etwa 0,8 bis 1,5 Halogenatome pro Polyolefinmolekül beträgt.

6. Verfahren nach Anspruch 5, dadurch gekennzeichnet, daß als Polyolefin Polyisobutene oder Polypropylene verwendet werden.

7. Verfahren nach einem der Ansprüche 5 und 6, dadurch gekennzeichnet, daß das halogenierte Polyolefin aus chloriertem Polyisobuten besteht.

8. Verfahren nach einem der Ansprüche 1 bis 7, dadurch gekennzeichnet, daß das Anhydrid der ungesättigten Carbonsäure aus Maleinsäureanhydrid besteht.

**Claims**

1. A process for manufacturing an alkenyl dicarboxylic acid anhydride, in which at least one halogenated polyolefine and at least one unsaturated dicarboxylic acid anhydride are reacted in substantially stoichiometric proportions, characterized in that it is effected in the presence of a minor proportion of at least one organic compound selected from halogenoquinones, cyanoquinones, halogenomaleic anhydrides and polycyanoolefins, at a temperature of 150—200" C.

2. A process according to claim 1, characterized in that said organic compound is dichloromaleic anhydride.

3. A process according to one of claim 1 and 2, characterized in that said organic compound is used in a molar proportion of 0.5 to 5% with respect to the quantity of unsaturated dicarboxylic acid anhydride involved.

4. A process according to one of claims 1 to 3, characterized in that it is effected at a temperature of 170—185° C.

5. A process according to one of claims 1 to 4, characterized in that said halogenated polyolefin is obtained by halogenation with chlorine, bromine, or iodine of a polyolefin of molecular weight from 200 to 3000, the halogenation rate being from about 0.8 to 1.5 halogen atom per molecule of polyolefin.

6. A process according to claim 5, characterized in that said polyolefin is selected from polyisobutenes and polypropylenes.

7. A process according to one of claims 5 and 6, characterized in that said halogenated polyolefin is a chlorinated polyisobutene.

8. A process according to one of claims 1 to 7, characterized in that said unsaturated dicarboxylic acid anhydride is maleic anhydride.